# EUROPÄISCHE PATENTANMELDUNG

(11) **EP 4 717 234 A1**
(43) Veröffentlichungstag der Anmeldung: **01.04.2026**
(21) Anmeldenummer: 25203803.9
(22) Anmeldetag: 22.09.2025
(51) Int. Cl.: A61F 5/01

(54) **SPERRGELENK**

(30) Priorität: 25.09.2024 DE 102024127744
(71) Anmelder: Ottobock SE & Co. KGaA, 37115 Duderstadt (DE)
(72) Erfinder: Schwartz, Miclas, 37115 Duderstadt (DE)
(74) Vertreter: Gramm, Lins & Partner Patent- und Rechtsanwälte PartGmbB

(57) **Zusammenfassung**

Die Erfindung betrifft ein Sperrgelenk für eine orthopädietechnische Einrichtung, wobei das Sperrgelenk
- ein erstes Gelenkteil (2) mit
∘ einem ersten Anschlag (6), der eine erste Anschlagsfläche (8) aufweist, und
∘ einem zweiten Anschlag (14), der eine zweite Anschlagsfläche (16) aufweist und in eine Freigabestellung und eine Sperrstellung bringbar ist, und

- ein zweites Gelenkteil (4) mit einer ersten Kontaktfläche (10) und einer zweiten Kontaktfläche (18) aufweist, das
∘ um eine Gelenksachse (20) schwenkbar am ersten Gelenkteil (2) angeordnet ist, wobei
∘ die Schwenkbewegung in eine erste Richtung durch den ersten Anschlag (6) begrenzt wird, wenn die erste Kontaktfläche (10) an der ersten Anschlagsfläche (8) anliegt, und wobei
o die Schwenkbewegung in eine zweite Richtung durch den zweiten Anschlag (14) begrenzt wird, wenn sich der zweite Anschlag (14) in der Sperrstellung befindet und die zweite Kontaktfläche (18) an der zweiten Anschlagsfläche (16) anliegt,

**dadurch gekennzeichnet, dass** die erste Kontaktfläche (10) und die zweite Kontaktfläche (18) an einem Trägerelement (12) angeordnet sind, das um eine Trägerachse schwenkbar an einem Grundkörper (22) des zweiten Gelenkteils (4) angeordnet ist.

## Beschreibung

Die Erfindung betrifft ein Sperrgelenk für eine orthopädietechnische Einrichtung, wobei das Sperrgelenk ein erstes Gelenkteil mit einem ersten Anschlag, der eine erste Anschlagsfläche aufweist, und einem zweiten Anschlag, der eine zweite Anschlagsfläche aufweist und in eine Freilaufstellung und eine Sperrstellung bringbar ist, und ein zweites Gelenkteil mit einer ersten Kontaktfläche und einer zweiten Kontaktfläche aufweist, das um eine Gelenksachse schwenkbar am ersten Gelenkteil angeordnet ist, wobei die Schwenkbewegung in eine erste Richtung durch den ersten Anschlag begrenzt wird, wenn die erste Kontaktfläche an der ersten Anschlagsfläche anliegt, und wobei die Schwenkbewegung in eine zweite Richtung durch den zweiten Anschlag begrenzt wird, wenn sich der zweite Anschlag in der Sperrstellung befindet und die zweite Kontaktfläche an der zweiten Anschlagsfläche anliegt.

Derartige Sperrgelenke sind aus dem Stand der Technik seit langem bekannt und werden in der Regel verwendet, um zwei Elemente der orthopädietechnischen Einrichtung miteinander zu verbinden. Dazu wird ein Element der orthopädietechnischen Einrichtung, beispielswiese ein Oberschenkelelement zum Anlegen an einen Oberschenkel, mit einem der beiden Gelenkteile verbunden. Ein anderes Element der orthopädietechnischen Einrichtung, beispielsweise ein Unterschenkelelement zum Anlegen an einen Unterschenkel wird mit dem anderen Gelenkteil verbunden. Gelenke dieser Art können beispielsweise in Knieorthesen verwendet werden. Derartige Orthesen stützen das Kniegelenk vor Überbelastung beispielsweise bei einer Lähmung der das Knie stabilisierenden Muskulatur und können das Kniegelenk, an dem sie angeordnet sind, ruhigstellen und sichern. Dazu wird der zweite Anschlag in die Sperrstellung gebracht, sodass die Schwenkbewegung der beiden Gelenkteile relativ zueinander sowohl in die erste Richtung als auch in die zweite Richtung begrenzt. Das Sperrgelenk ist dann gesperrt, wenn der erste Anschlag und der zweite Anschlag gleichzeitig an dem zweiten Gelenkteil anliegen und so eine Bewegung weder in die erste Richtung noch in die zweite Richtung möglich ist. Die erste Richtung ist der zweiten Richtung entgegengerichtet. Der erste Anschlag liegt an dem zweiten Gelenkteil an, wenn die erste Anschlagsfläche an der ersten Kontaktfläche anliegt. Der zweite Anschlag liegt an dem zweiten Gelenkteil an, wenn die zweite Anschlagsfläche an der zweiten Kontaktfläche anliegt. In diesem Zustand verhindert das Sperrgelenk eine Bewegung der beiden Gelenkteile zueinander. Als Teil einer Gelenkorthese, beispielsweise einer Knieorthese wird das erste Gelenkteil mit einem ersten Körperteil und das zweite Gelenkteil mit einem zweiten Körperteil verbunden, so dass das Sperrgelenk ein zwischen dem ersten Körperteil und dem zweiten Körperteil befindliches Gelenk des menschlichen Körpers überspannt.

Da eine Bewegung des Sperrgelenkes in diesem Zustand nicht erfolgen soll, ist es enormen Kräften und Momenten ausgesetzt. Nachteilig ist, dass alle verwendeten Bauteile mit einem Spiel behaftet sind. Dies kann eine fertigungsbedingte Abweichung von den Normmaßen des jeweiligen Bauteils sein oder beispielsweise durch Verschleiß hervorgerufen werden. Die Sicherheit des Sperrgelenkes wird dadurch in der Regel nicht beeinflusst, es kann aber zu Geräuschentwicklung und einem Gefühl der Unsicherheit für den Benutzer kommen. Wechselt beispielsweise im Verlauf eines Schrittzyklus die auf die Gelenkteile wirkende Kraft und/oder ein entsprechend wirkendes Drehmoment das Vorzeichen, kommt es durch das vorhandene Spiel zwischen den Bauteilen des Sperrgelenks zu einer oft nur sehr kleinen Bewegung des ersten Gelenkteils relativ zu dem zweiten Gelenkteil. Nach dieser kleinen Bewegung schlägt das zweite Gelenkteil an einen der beiden Anschläge an, wobei der Anschlag von der Schwenkrichtung und damit von der wirkenden Kraft oder dem wirkenden Moment abhängt. Beim Anschlagen entsteht dann ein Geräusch.

Um dieses Spiel zu vermeiden, werden die Anschläge mit den daran angeordneten Anschlagsflächen in der Regel individuell hergestellt. Die Anschlagsfläche wird mechanisch nachbearbeitet, bis sie möglichst gut an die jeweilige Kontaktfläche, mit der sie in Kontakt kommen soll, angepasst ist. Dies ist zeitaufwendig und damit kostenintensiv. Zudem führen Fehler bei der Nachbearbeitung dazu, dass einmal zu viel entferntes Material nicht wieder auf die Anschlagsfläche aufgebracht werden kann. Es muss also ein neuer Anschlag verwendet und die Arbeit erneut begonnen werden.

Bei einer bilateralen Beinversorgung, bei der ein Sperrgelenk medial und ein Sperrgelenk lateral an der versorgten Extremität, beispielsweise an einem Kniegelenk, angeordnet ist, besteht zudem das Risiko, dass bei einem Sperrgelenk ein Gelenkteil bereits im Anschlag und die Sperre sich schon in der Sperrposition befindet und das gegenüberliegende Sperrgelenk noch geöffnet ist. Es besteht das Risiko, dass die Sperrklinken der beiden Sperrgelenke nicht synchron einrasten.

Aus der DE 10 2022 134 430 A1 ist ein Sperrgelenk gemäß dem Oberbegriff des Anspruchs 1 bekannt, bei dem das Gelenk einen Exzenter aufweist, durch den eine der vier Kontaktflächen, also die erste Anschlagsfläche, die zweite Anschlagsfläche, die erste Kontaktfläche oder die zweite Kontaktfläche verschiebbar ist. Dadurch wird das Sperrgelenk derart einstellbar, dass es in der gesperrten Stellung, in der keine Bewegung des Gelenks möglich ist, bei wechselnder Belastung nicht zu den nachteiligen "klick"-Geräuschen kommen kann, da kein Spiel im Gelenk vorhanden ist.

Nachteilig ist jedoch, dass die Sperrposition, also der Winkel des Gelenks, wenn es gesperrt ist und keine Bewegung zwischen dem ersten Gelenkteil und dem zweiten Gelenkteil möglich ist, nicht eingestellt werden kann. Die Sperrposition ist dadurch festgelegt, dass die erste Kontaktfläche an der ersten Anschlagsfläche und die zweite Kontaktfläche an der zweiten Anschlagsfläche anliegt. In diesem Zustand ist eine Bewegung der beiden Gelenkteile relativ zueinander nicht möglich und auch nicht erwünscht.

Das erste Gelenkteil und das zweite Gelenkteil beinhalten entweder ein Element einer orthopädietechnischen Einrichtung oder weisen eine Befestigungseinrichtung auf, an der ein solches Element, beispielsweise eine Schiene oder eine Schale zum Anordnen an ein Körperteil, befestigt werden kann. In einem einfachen Ausführungsbeispiel ist eine solche Befestigungseinrichtung in Form von zwei oder mehr Löchern ausgebildet, durch die entsprechende Elemente, beispielsweise Stifte, Schrauben oder Bolzen hindurchgesteckt werden. In der Position nehmen die beiden Elemente der orthopädietechnischen Einrichtung einen Winkel zueinander ein, der als Sperrwinkel bezeichnet wird.

Der Erfindung liegt die Aufgabe zugrunde, ein Sperrgelenk gemäß dem Oberbegriff des Anspruchs 1 so weiterzuentwickeln, dass der Sperrwinkel einstellbar ist.

Die Erfindung löst die gestellte Aufgabe durch ein Sperrgelenk gemäß dem Oberbegriff des Anspruchs 1, das sich dadurch auszeichnet, dass die erste Kontaktfläche und die zweite Kontaktfläche an einem Trägerelement angeordnet sind, das um eine Trägerachse schwenkbar an einem Grundkörper des zweiten Gelenkteils angeordnet ist.

Das Trägerelement ist in verschiedenen Orientierungen relativ zu dem Grundkörper arretierbar. In diesem Zustand ist eine Verschwenkung des Trägerelementes relativ zu dem zweiten Gelenkteil um die Trägerachse nicht möglich.

Vorzugsweise sind die beiden Kontaktflächen an einem gemeinsamen Trägerelement angeordnet. Die beiden Kontaktelemente können mit dem Trägerelement relativ zu dem Grundkörper des zweiten Gelenkteils bewegt, insbesondere um die Trägerachse verschwenkt werden. Dies ist auch dann möglich, wenn sich das Gelenk in der Sperrposition befindet und die beiden Kontaktflächen an der jeweiligen Anschlagsflächen anliegen. Das Trägerelement kann in dieser Position nicht relativ zu dem ersten Gelenkteil bewegt werden. Eine solche Bewegung wird in eine Richtung den Kontakt zwischen der ersten Kontaktfläche und der ersten Anschlagsfläche und in die entgegengesetzte Richtung durch den Kontakt zwischen der zweiten Kontaktfläche und der zweiten Anschlagsfläche verhindert. Die Schwenkbewegung zwischen dem Trägerelement und dem Grundkörper des zweiten Gelenkteils ist jedoch weiterhin möglich.

Daher ist auf diese Weise auch eine Bewegung des Grundkörpers des zweiten Gelenkteils, an dem sich das Element der orthopädietechnischen Einrichtung befindet, relativ zu dem ersten Gelenkteil, an dem sich ein weiteres Element der orthopädietechnischen Einrichtung befindet, möglich.

Wenn die beiden Kontaktflächen an einem gemeinsamen Trägerelement befestigt sind, sind sie nur gemeinsam bewegbar, so dass sich ihre Position und Orientierung relativ zueinander nicht ändert. Insbesondere bleibt der Abstand der beiden Kontaktflächen zueinander konstant, auch wenn das Trägerelement relativ zu dem Grundkörper des zweiten Gelenkteils bewegt wird. Dies bedeutet, dass eine Einstellung der beiden Anschläge, wie aus der bereits genannten
DE 10 2022 134 430 A1 bekannt, bestehen bleibt und auch die dadurch erreichte oder erreichbare Spielfreiheit bestehen bleibt.

Vorzugsweise sind die Trägerachse und die Gelenksachse identisch. Wird in diesem Fall das Trägerelement relativ zum Grundkörper bewegt, während sich das Schwergelenk in der Sperrstellung befindet, kommt es nicht zu einer Relativbewegung der beiden Kontaktflächen des Trägerelementes zu den beiden Anschlagsflächen des ersten Gelenkteils. Insbesondere bleibt die Orientierung der Kontaktflächen relativ zu den Anschlagsflächen in diesem Falle gleich, sodass auch der Kontaktbereich, also der Bereich der jeweiligen Flächen, an denen der Kontakt erfolgt, unverändert bleibt.

In einer bevorzugten Ausgestaltung verfügt das zweite Gelenkteil oder das Trägerelement über ein Betätigungselement, durch dessen Betätigen das Trägerelement um die Trägerachse relativ zu dem Grundkörper verschwenkt wird. Das Betätigungselement ist vorzugsweise so ausgebildet, dass eine Schwenkbewegung des Trägerelementes relativ zu dem Grundkörper des zweiten Gelenkteils nur dann stattfinden kann, wenn das Betätigungselement betätigt wird. Wird das Betätigungselement nicht betätigt, kann auch keine Schwenkbewegung des Trägerelementes relativ zu dem Grundkörper des zweiten Gelenkteils stattfinden. In einigen Ausgestaltungen verfügt das Sperrgelenk über eine Verriegelungseinrichtung, beispielsweise zwei miteinander in Eingriff stehende Formschlusselemente, die im verriegelten Zustand eine Schwenkbewegung des Trägerelementes relativ zum Grundkörper des zweiten Gelenkteils verhindert. Durch Betätigen des Betätigungselement wird die Verriegelungseinrichtung gelöst, also aus einem verriegelten Zustand in einen geöffneten Zustand überführt. Weist die Verriegelungseinrichtung die oben bereits genannten Formschlusselemente auf, werden diese durch das Betätigen des Betätigungselementes außer Eingriff gebracht. Dadurch wird die Schwenkbewegung zwischen dem Trägerelement und dem Grundkörper des zweiten Gelenkteils ermöglicht. Das Betätigungselement kann beispielsweise als Druckknopf ausgebildet sein, auf den eine Kraft, beispielsweise ein Druck, ausgeübt werden muss, um es zu betätigen.

Vorteilhafterweise weist das Trägerelement oder das zweite Gelenkteil einen Vorsprung auf und das Betätigungselement verfügt über zwei Einzelelemente, die aneinander gegenüberliegenden Seiten des Vorsprungs angeordnet sind und vorzugsweise an diesen Seiten anliegen. Wenn das zweite Gelenkteil das Betätigungselement aufweist, ist das Trägerelement mit dem Vorsprung versehen. Weist das zweite Gelenkteil den Vorsprung auf, ist das Trägerelement mit dem Betätigungselement versehen. Ein Betätigungselement und ein Vorsprung, der an dem zweiten Gelenkteil angeordnet ist, ist vorzugsweise am Grundkörper des zweiten Gelenkteiles angeordnet. Die Einzelelemente sind vorzugsweise als Druckelemente ausgebildet, über die ein Druck auf den Vorsprung des Trägerelementes ausgeübt werden kann. Die Einzelelemente sind vorzugsweise als Einlegekeile, Pass-Stifte oder Schrauben, insbesondere als Gewindestifte ausgebildet. Um in dieser Ausgestaltung das Trägerelement relativ zum Grundkörper des zweiten Gelenkteils zu Verschwenken, wird zunächst eines der Einzelelemente so bewegt, dass es nicht mehr am Vorsprung des Trägerelementes anliegt. Dies kann beispielsweise dadurch geschehen, dass ein als Schraube, insbesondere Gewindestift, ausgebildetes Einzelelement, das in einem mit einem Innengewinde ausgebildeten Kanal angeordnet ist, innerhalb dieses Kanals so gedreht wird, dass es sich von der Seite des Vorsprungs entfernt. Danach kann das zweite Einzelelement, das mit der gegenüberliegenden Seite des Vorsprungs in Kontakt steht, so bewegt werden, dass es einen Druck auf den Vorsprung des Trägerelementes ausübt und durch diese Kraft ein Drehmoment auf das Trägerelement ausgeübt wird, durch das das Trägerelement relativ zum Grundkörper des zweiten Gelenkteils verschwenkt wird. Ist die gewünschte Position erreicht, kann das erste Einzelelement wieder in die entgegengesetzte Richtung bewegt werden, um es wieder in Kontakt mit dem Vorsprung des Trägerelementes zu bringen. Dadurch wird gewährleistet, dass kein Spiel vorhanden ist, dass zu den unerwünschten Geräuschen führt.

Vorzugsweise sind die Druckelemente in Richtung auf den Vorsprung federbelastet. Dadurch wird erreicht, dass das Gelenk bei ausreichend großen Drehmomenten federnd nachgibt, wobei der dadurch erlaubte Bewegungsspielraum durch die verwendeten Federelemente, die die Federbelastung hervorrufen, bestimmt wird. Je härter die Federelemente sind, desto größer müssen die wirkenden Drehmomente sein, um das Gelenk zum Einfedern zu bringen. Je kürzer die Federelemente sind, desto kürzer ist der zum Einfedern zur Verfügung stehende Federweg. Durch die Federelemente wird zudem erreicht, dass das Sperrgelenk nach dem Wegfall der Drehmomente, die zum Einfedern geführt haben, wieder in die Ausgangsposition zurückbewegt wird.

In einer bevorzugten Ausgestaltung weist das Gelenk eine Hydraulikanordnung auf, die eingerichtet ist, die Einzelelemente zu verschieben. Die Hydraulikanordnung verfügt dabei vorzugsweise über zwei Hydraulikkammern, die miteinander fluidtechnisch verbunden sind. Dazu ist beispielsweise eine Fluidverbindung vorhanden. Die Hydraulikanordnung weist vorzugsweise zudem ein Ventil auf, durch das die Fluidleitung verschlossen und geöffnet werden kann. Wird das Ventil geöffnet, ist es möglich, Hydraulikfluid durch die Fluidverbindung von einer Hydraulikkammer in die jeweils andere Hydraulikkammer zu leiten. Die Einzelelemente sind vorzugsweise Hydraulikkolben oder mit diesen verbundene Bauteile. Wird Fluid von einer Kammer in die andere Kammer geleitet, wird das Volumen der ersten Kammer reduziert und gleichzeitig das Volumen der zweiten Kammer entsprechend vergrößert. Dies hat bei optimaler Ausgestaltung der Kammern zur Folge, dass beide Einzelelemente um die gleiche Strecke verschoben werden. Da die Einzelelemente mit dem Vorsprung in Kontakt sind, wird auch der Vorsprung verschoben, so dass Trägerelement und zweites Gelenkteil relativ zueinander um die Trägerachse verschwenkt werden. Ist die gewünschte Orientierung von Trägerelement und zweitem Gelenkteil erreicht, wird vorzugsweise das Ventil und damit die Fluidverbindung geschlossen. Es ist daher nicht möglich, weiteres Fluid zwischen den Hydraulikkammern auszutauschen, so dass auch eine weitere Verschwenkung des Trägerelementes relativ zu dem zweiten Gelenkteil nicht mehr möglich ist.

Vorzugsweise ist das Trägerelement spielfrei an dem Grundkörper angeordnet. Besonders bevorzugt ist das Trägerelement in jeder Schwenkposition spielfrei an dem Grundkörper angeordnet.

Bevorzugt ist die Orientierung des Trägerelementes relativ zu dem zweiten Gelenkteil, in der diese beiden Elemente relativ zueinander arretierbar sind, stufenlos einstellbar.

Mithilfe der beigefügten Zeichnungen wird nachfolgend ein Ausführungsbeispiel der vorliegenden Erfindung näher erläutert. Es zeigen:
Figuren 1 bis 3 - ein Sperrgelenk gemäß einem Ausführungsbeispiel der vorliegenden Erfindung in drei unterschiedlichen Sperrpositionen,
Figur 4 - das Sperrgelenk im entsperrten Zustand und
Figuren 5 bis 8 - Darstellungen von Sperrgelenken gemäß weiterer Ausführungsbeispiele der vorliegenden Erfindung.

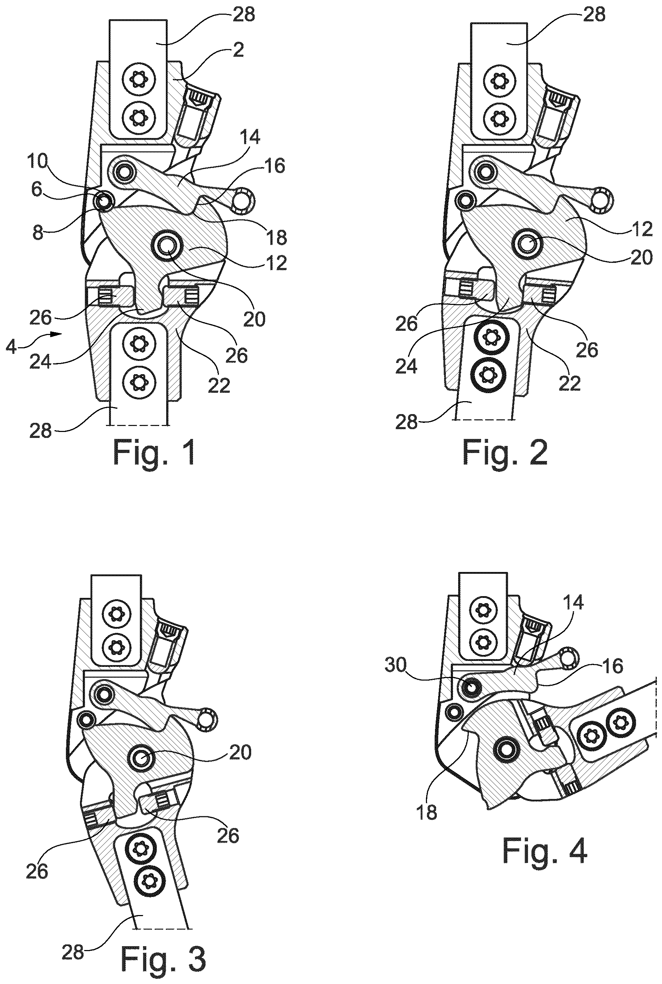

Figur 1 zeigt eine schematische Darstellung eines Sperrgelenkes gemäß einem Ausführungsbeispiel der vorliegenden Erfindung. Es verfügt über ein erstes Gelenkteils 2 und ein zweites Gelenkteil 4. Am ersten Gelenkteil 2 befindet sich ein erster Anschlag 6 mit einer ersten Anschlagfläche 8, an der in der gezeigten Position des Sperrgelenkes eine erste Kontaktfläche 10 anliegt, die an einem Trägerelement 12 angeordnet ist. Das erste Gelenkteils 2 verfügt zudem über einen zweiten

Anschlag 14, der eine zweite Anschlagfläche 16 aufweist, an der in der gezeigten Position des Sperrgelenkes eine zweite Kontaktfläche 18 anliegt.

Das erste Gelenkteil 2 und das zweite Gelenkteil 4 sind um eine Gelenksachse 20 relativ zueinander schwenkbar angeordnet. Das Trägerelement 12 ist an einem Grundkörper 22 des zweiten Gelenkteils 4 angeordnet. Das Trägerelement 12 verfügt über einen Vorsprung 24, an dessen zwei gegenüberliegenden Seiten jeweils ein Einzelelement 26 angeordnet ist. Bei der Einzelelemente 26 sind im gezeigten Ausführungsbeispiel als Gewindestifte ausgebildet und verfügen über ein nicht dargestelltes Außengewinde. Sie befinden sich in jeweils einer Bohrung, die mit einem entsprechenden Innengewinde ausgestattet ist. Jedes der Einzelelemente 26 verfügt über ein Formschlusselemente, in das ein entsprechendes Werkzeug eingreifen kann, um die Einzelelemente 26 zu drehen und so tiefer in die Bohrung hinein oder aus der Bohrung heraus zu bewegen. Auf diese Weise lässt sich eine Kraft auf den Vorsprung 24 des Trägerelementes 12 ausüben, die zu einer Verschwenkung des Trägerelementes 12 relativ zum Grundkörper 22 des zweiten Gelenkteils 4 führt.

Figur 1 zeigt das Sperrgelenk in einer ersten Sperrposition. Sowohl am ersten Gelenkteil 2 also vom zweiten Gelenkteil 4 ist schematisch jeweils ein Element 28 der orthopädietechnischen Einrichtung dargestellt, deren Teil das Sperrgelenk ist oder zumindest sein kann. Man erkennt in Figur 1, dass die beiden Elemente 28 parallel zueinander ausgerichtet sind. In dieser Stellung ist der Sperrwinkel folglich 0°.

Figur 2 zeigt das Gelenk aus Figur 1 in einer anderen Sperrposition. Man erkennt, dass die beiden Einzelelemente 26 bewegt wurden. Das in Figur 2 links dargestellte Einzelelement 26 ist tiefer in seine Bohrung hinein bewegt worden. Das in Figur 2 rechts dargestellte Einzelelement 26 ist entsprechend weiter aus seiner Bohrung heraus bewegt worden. Wie auch in Figur 1 liegen beide Einzelelemente 26 an jeweils einer Seite des Vorsprungs 24 des Trägerelementes 12 an. Es handelt sich daher um eine spielfreie Verbindung. Durch die Bewegung der beiden Einzelelemente 26 in den jeweiligen Bohrungen, die sich im Grundkörper 22 des zweiten Gelenkteils 4 befinden hat sich der Winkel zwischen den beiden Elementen 28 der orthopädietechnischen Einrichtung gegenüber dem Winkel aus Figur 1 verändert. Man erkennt das das unten dargestellte Element 28 im Uhrzeigersinn um die Gelenksachse 20 verschwenkt wurde.

Figur 3 zeigt das Gelenk in einer dritten Sperrposition. Im Vergleich zu Figur 2 sind wieder die beiden Einzelelemente 26 bewegt worden. Das in Figur 3 rechts dargestellte Einzelelement 26 ist tiefer in seine Bohrung hineinverschoben worden. Das in Figur 3 links dargestellte Einzelelement 26 ist aus seiner Bohrung heraus bewegt worden. Wie auch in Figur 1 und 2 liegen beide Einzelelemente 26 am Vorsprung 24 des Trägerelementes 12 an, sodass auch hier kein Spiel vorhanden ist. Wieder ist die Position der beiden Elemente 28 der orthopädietechnischen Einrichtung verändert worden. Im Vergleich zu Figur 1 ist das untere der beiden Elemente 28 gegen den Uhrzeigersinn um die Gelenksachse 20 verschwenkt worden.

Figur 4 zeigt das Gelenk in der entsperrten Position. Man erkennt, dass der zweite Anschlag 14 um eine Anschlagsachse 30 herum verschwenkt wurde. Die zweite Anschlagfläche 16 liegt daher nicht mehr an der zweiten Kontaktfläche 18 an und das Gelenk kann bewegt werden. Man erkennt insbesondere in Figur 4, dass das zweite Gelenkteil 4 relativ zum ersten Gelenkteil 2 um die Gelenksachse 20 herum verschwenkt wurde.

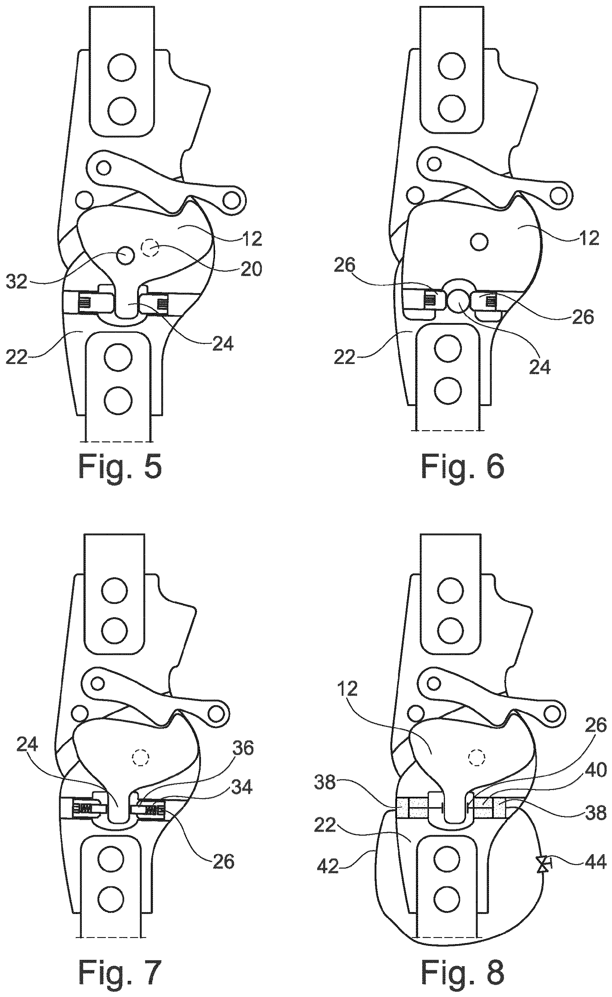

In den Figuren 5 bis 8 sind schematisch Sperrgelenke gemäß verschiedener Ausführungsformen der vorliegenden Erfindung dargestellt. Die Sperrgelenke unterscheiden sich hauptsächlich durch die Art, wie eine Bewegung der jeweiligen Trägerelemente 12 zu den entsprechenden zweiten Gelenkteilen 4 ermöglicht wird.

In Figur 5 weist das Trägerelement 12 den Vorsprung 24 auf. Die Gelenksachse 20 ist in Figur 5 als gestrichelter Kreis dargestellt. Das Gelenk weist eine davon beabstandete Trägerachse 32 auf, um die das Trägerelement 12 relativ zu dem zweiten Gelenkteil 4 verschwenkt werden kann.

Figur 6 zeigt eine Ausführungsform, bei der der Vorsprung 24 an dem Grundkörper 22 des zweiten Gelenkteils 4 angeordnet ist. Er ist in Form eines aus der Zeichenebene heraus gerichteten Pins oder Stift ausgebildet. Die Einzelelemente 26 sind entsprechend am Trägerelement 12 positioniert.

Figur 7 zeigt eine Ausführungsform, bei der die Einzelelemente 26 am Grundkörper 22 des zweiten Gelenkteils angeordnet und federbelastet ausgebildet sind. Sie beinhalten jeweils eine Feder 34 und einen durch die Feder 34 federbelasteten Stift 36, der den eigentlichen Kontakt zu dem Vorsprung 12 herstellt.

In Figur 8 ist eine Ausführungsform dargestellt, die ein Hydrauliksystem mit zwei Hydraulikkammern 38 aufweist. Die eigentlichen Einzelelemente 26, die den Kontakt zu dem Vorsprung 24 herstellen, sind mit jeweils einem Kolben 40 der beiden Hydraulikkammern verbunden. Die beiden Hydraulikkammern 38 sind durch eine nur schematisch dargestellte Fluidverbindung 42 miteinander verbunden, in der sich ein Ventil 44 befindet. Ist das Ventil 44 geöffnet, kann Hydraulikfluid von einer der beiden Hydraulikkammern 38 in die jeweils andere Hydraulikkammer 38 fließen. Dann wird das Trägerelement 12 relativ zu dem Grundkörper 22 des zweiten Gelenkteils 4 verschwenkt. Ist hingegen das Ventil 44 geschlossen, ist auch die Fluidverbindung 42 geschlossen und eine Bewegung des Trägerelementes 12 relativ zu dem Grundkörper 22 ist unterbunden.

### Bezugszeichenliste

2 erstes Gelenkteil
4 zweites Gelenkteil
6 erster Anschlag
8 erste Anschlagsfläche
10 erste Kontaktfläche
12 Trägerelement
14 zweiter Anschlag
16 zweite Anschlagsfläche
18 zweite Kontaktfläche
20 Gelenksachse
22 Grundkörper
24 Vorsprung
26 Einzelelemente
28 Element
30 Anschlagsachse
32 Trägerachse
34 Feder
36 Stift
38 Hydraulikkammer
40 Kolben
42 Fluidverbindung
44 Ventil

## Patentansprüche

1. Sperrgelenk für eine orthopädietechnische Einrichtung, wobei das Sperrgelenk
- ein erstes Gelenkteil (2) mit
∘ einem ersten Anschlag (6), der eine erste Anschlagsfläche (8) aufweist, und
∘ einem zweiten Anschlag (14), der eine zweite Anschlagsfläche (16) aufweist und in eine Freigabestellung und eine Sperrstellung bringbar ist, und
- ein zweites Gelenkteil (4) mit einer ersten Kontaktfläche (10) und einer zweiten Kontaktfläche (18) aufweist, das
∘ um eine Gelenksachse (20) schwenkbar am ersten Gelenkteil (2) angeordnet ist, wobei
∘ die Schwenkbewegung in eine erste Richtung durch den ersten Anschlag (6) begrenzt wird, wenn die erste Kontaktfläche (10) an der ersten Anschlagsfläche (8) anliegt, und wobei
∘ die Schwenkbewegung in eine zweite Richtung durch den zweiten Anschlag (14) begrenzt wird, wenn sich der zweite Anschlag (14) in der Sperrstellung befindet und die zweite Kontaktfläche (18) an der zweiten Anschlagsfläche (16) anliegt,
**dadurch gekennzeichnet, dass** die erste Kontaktfläche (10) und die zweite Kontaktfläche (18) an einem Trägerelement (12) angeordnet sind, das um eine Trägerachse schwenkbar an einem Grundkörper (22) des zweiten Gelenkteils (4) angeordnet ist.

2. Sperrgelenk nach Anspruch 1, **dadurch gekennzeichnet, dass** die Trägerachse und die Gelenksachse (20) identisch sind.

3. Sperrgelenk nach Anspruch 1 oder 2, **dadurch gekennzeichnet, dass** das zweite Gelenkteil (4) oder das Trägerelement (12) ein Betätigungselement aufweist, durch dessen Betätigen das Trägerelement (12) um die Trägerachse relativ zu dem Grundkörper (22) verschwenkt wird.

4. Sperrgelenk nach Anspruch 3, **dadurch gekennzeichnet, dass** das Trägerelement (12) oder das zweite Gelenkteil (4) einen Vorsprung (24) aufweist und das Betätigungselement zwei Einzelelemente (26) aufweist, die an zwei gegenüberliegenden Seiten des Vorsprunges (24) angeordnet sind.

5. Sperrgelenk nach Anspruch 4, **dadurch gekennzeichnet, dass** die Einzelelemente (26) als Druckelemente, beispielsweise als Einlegekeile, Pass-Stifte, Schrauben, insbesondere als Gewindestifte ausgebildet sind.

6. Sperrgelenk nach Anspruch 5, **dadurch gekennzeichnet, dass** die Druckelemente in Richtung auf den Vorsprung (24) federbelastet sind.

7. Sperrgelenk nach Anspruch 4 oder 5, **dadurch gekennzeichnet, dass** das Gelenk eine Hydraulikanordnung aufweist, die eingerichtet ist, die Einzelelemente (26) zu verschieben.

8. Sperrgelenk nach einem der vorstehenden Ansprüche, **dadurch gekennzeichnet, dass** das Trägerelement (12) spielfrei an dem Grundkörper (22) angeordnet ist.

9. Sperrgelenk nach Anspruch 8, **dadurch gekennzeichnet, dass** das Trägerelement (12) in jeder Schwenkposition spielfrei an dem Grundkörper (22) angeordnet ist.

10. Sperrgelenk nach einem der vorstehenden Ansprüche, **dadurch gekennzeichnet, dass** das Trägerelement (12) stufenlos an dem Grundkörper (22) arretierbar ist.
